# EUROPEAN PATENT SPECIFICATION

(11) **EP 1 222 280 B1**
(45) Date of publication and mention of the grant of the patent: **06.07.2005**
(21) Application number: 00978103.0
(22) Date of filing: 20.10.2000
(51) Int. Cl.: C12N 15/34, C07K 14/01, C07K 16/08, A61K 39/12, A61K 48/00

(54) **APOPTOSIS INDUCING PROTEINACEOUS SUBSTANCE**
APOPTOSE INDUZIERENDE PROTEINESUBSTANZ
SUBSTANCE PROTEIQUE INDUISANT L'APOPTOSE

(30) Priority: 21.10.1999 EP 99203467
(43) Date of publication of application: 17.07.2002
(73) Proprietor: Leadd B.V., 2333 AL Leiden (NL)
(72) Inventor: NOTEBORN, Mathieu, Hubertus, Maria, NL-2352 EH Leiderdorp (NL); WEISS, Bertram, 14195 Berlin (DE)
(74) Representative: Prins, Adrianus Willem, Mr. Ir.
(86) International application number: PCT/NL2000/000767
(87) International publication number: WO 2001/029226

(56) References cited:
- EP-A- 0 924 296
- NISHIZAWA T. ET AL.: "Non-B, non-C, non-G hepatitis virus gene DNA sequence." GENESEQ DATABASE ; ACCESSION NUMBER X18959, 13 May 1999 (1999-05-13), XP002136316
- OKAMOTO HIROAKI ET AL: "The entire nucleotide sequence of a TT virus isolate from the United States (TUS01): Comparison with reported isolates and phylogenetic analysis." VIROLOGY JULY 5, 1999, vol. 259, no. 2, 5 July 1999 (1999-07-05), pages 437-448, XP002136317 ISSN: 0042-6822 -& OKAMOTO H. ET AL.: "TT virus genotype 1a DNA, complete genome." EMBL DATABASE ; ACCESSION NUMBER AB017610, 14 August 1999 (1999-08-14), XP002136318
- OKAMOTO H. ET AL: "Molecular cloning and characterization of a novel DNA virus (TTV) associated with posttransfusion hepatitis of unknown etiology" HEPATOLOGY RESEARCH_(_HEPATOL. RES._), 10/1 (1-16), XP000892167 Ireland -& OKAMOTO H. ET AL.: " TT virus genes for ORF2 and ORF1, complete cds." EMBL DATABSE; ACCESSION NUMBER AB008394, 13 March 1998 (1998-03-13), XP002136319
- ROULSTON A. ET AL: "Viruses and apoptosis" ANNUAL REVIEW OF MICROBIOLOGY_(_ANNU. REV. MICROBIOL._), 1999, 53/- (577-628), XP000892185 United States
- DATABASE MEDLINE [Online] US NATIONAL LIBRARY OF MEDICINE (NLM), BETHESDA, MD, US; HASEBE C ET AL: "[Histological findings of TTV-positive non-B non-C chronic hepatitis patients]" retrieved from DIALOG Database accession no. 10023599 XP002172308 & NIPPON RINSHO, JUN 1999, 57 (6) P1350-5, JAPAN

## Description

The invention relates to the field of apoptosis.

Apoptosis is an active and programmed physiological process for eliminating superfluous, altered or malignant cells (Earnshaw, 1995, Duke et al., 1996).

Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with DNA- staining agents, such as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis, e.g. in cancer development and auto-immune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various Lotem, 1997).

For such cancers (representing more than half of the tumors) alternative anti-tumor therapies are under development based on induction of apoptosis independent of p53 (Thompson 1995, Paulovich et al., 1997). For this one has to search for the factors involved in induction of apoptosis thatdo not need p53 and/or cannot be blocked by anti-apoptotic activities, such as Bcl-2 or Bcr-abl-like ones. These factors might be part of a distinct apoptosis pathway or being (far) downstream to the apoptosis inhibiting compounds.

Apoptin (or VP3, the terms may be used interchangeable herein) is a small protein derived from chicken anemia virus (CAV; Noteborn and De Boer, 1996, Noteborn et al., 1991, Noteborn et al., 1994; 1998a), which can induce apoptosis in human malignant and transformed cell lines (the terms tumorigenic, transformed and malignant will be used interchangeable herein), but not in untransformed human cell cultures. *In vitro*, Apoptin fails to induce apoptosis in normal lymphoid, dermal, epidermal, endothelial and smooth-muscle cells. However, when normal cells are transformed they become susceptible to apoptosis by Apoptin. Long-term expression of Apoptin in normal human fibroblasts revealed that Apoptin has no toxic or transforming activity in these cells (Danen-van Ooschot, 1997 and Noteborn, 1996).

In normal cells, Apoptin was found predominantly in the cytoplasm, whereas in transformed or malignant cells i.e. characterized by hyperplasia, metaplasia or dysplasia, it was located in the nucleus, suggesting that the localization of Apoptm is related to its activity (Danen-van Oorschot et al. 1997).

Apoptin-induced apoptosis occurs in the absence of functional p53 (Zhuang et al., 1995a), and cannot be blocked by Bcl-2, Bcr-abl (Zhuang et al., 1995), the Bcl-2-associating protein BAG-1 and not by the caspase-inhibitor cowpox protein CrmA (Danen-Van Oorschot, 1997a, Noteborn, 1996). Therefore, Apoptin is a particularly useful therapeutic compound for the destruction of tumor cells, or other hyperplasia, metaplasia or dysplasia which have become resistant to (chemo)-therapeutic induction of apoptosis, due to for example the resistant to (chemo)-therapeutic induction of apoptosis, due to for example the lack of functional p53 and (over)-expression of Bcl-2 and other apoptosis-inhibiting agents (Noteborn and Pietersen, 1998).

It appears, that even pre-malignant, minimally transformed cells, are sensitive to the death-inducing effect of Apoptin. In addition, Noteborn and Zhang (1998) have shown that Apoptin-induced apoptosis can be used as diagnosis of cancer-prone cells and treatment of cancer-prone cells.

The fact that Apoptin does not induce apoptosis in normal human cells, at least not *in vitro*, implies that there would be little or no toxic effect of Apoptin treatment *in vivo*. Noteborn and Pietersen (1998) and Pietersen et al. (1999) have provided evidence that adenovirus expressed Apoptin does not have an acute toxic effect *in vivo*. In addition, in nude mice it was shown that Apoptin has a strong anti-tumor activity.

However, to further enlarge the array of therapeutic anti-cancer or anti-auto-immune-disease compounds available in the art, additional therapeutic compounds are desired that are designed to work alone, sequentially to, or jointly with Apoptin or other apoptosis inducing substances, especially in those cases wherein the tumor suppressor p53 is (partly) non-functional and or anti-apoptosis proteins such as Bcl-2 are over-expressed.

The invention provides novel therapeutic possibilities, for example novel combinatorial therapies or novel therapeutic compounds that can work alone, sequentially or jointly with Apoptin, especially in those cases wherein p53 is (partly) non-functional and/or Bcl-2 is over-expressed and/or functional caspase-3, an essential protease of the apoptotic machinery, is lacking.

In a first embodiment, the invention provides an apoptosis inducing protein, capable of providing apoptosis, alone or in combination with other apoptosis-inducing substances, such as Apoptin, said protein encoded by an isolated or recombinant nucleic acid or functional equivalent and/or fragment thereof derivable from a mammalian gyrovirus-like virus nucleic acid.

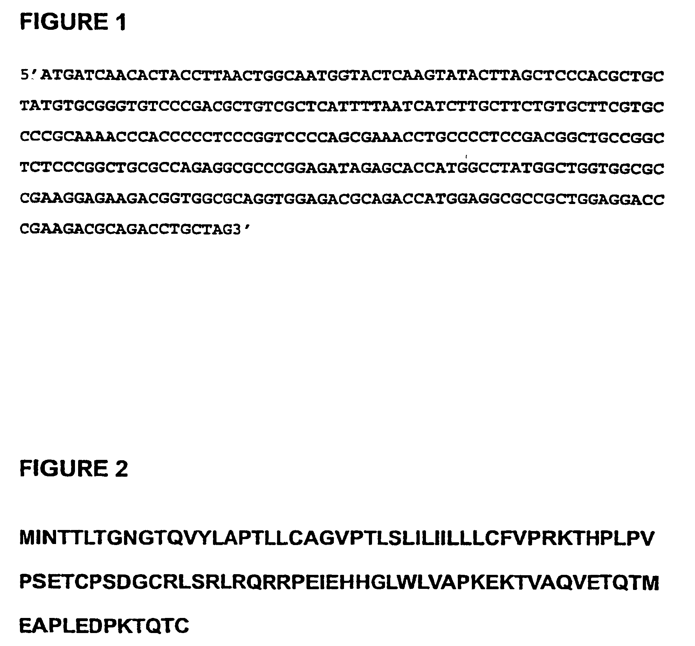

Mammalian gyrovirus-like viruses, which are circovirus-like single-stranded circular DNA viruses distinct from for example porcine circovirus or relatives thereof, have been found which surprisingly can be used to provide a substance providing apoptosis, thereby providing functional equivalent proteinaceous substances and/or fragments thereof having similar features as CAV-derived Apoptin or fragments thereof. In particular, the invention provides a protein according to the invention wherein said mammalian gyrovirus-like virus comprises a single-stranded circular DNA virus, such as for example a TTV-like virus, such as seen with the recently discovered novel human single-stranded circular DNA virus TTV (Charlton et al., 1998; Miyata et al., 1999). In a preferred embodiment, the invention provides a protein or functional fragment thereof, said substance encoded by an isolated or recombinant nucleic acid which is at least 60% homologous preferably at least 70%, more preferably at least 80%, even more preferably at least 90%, most preferably at least 95% to a TTV-like virus nucleic acid as shown in Figure 1.

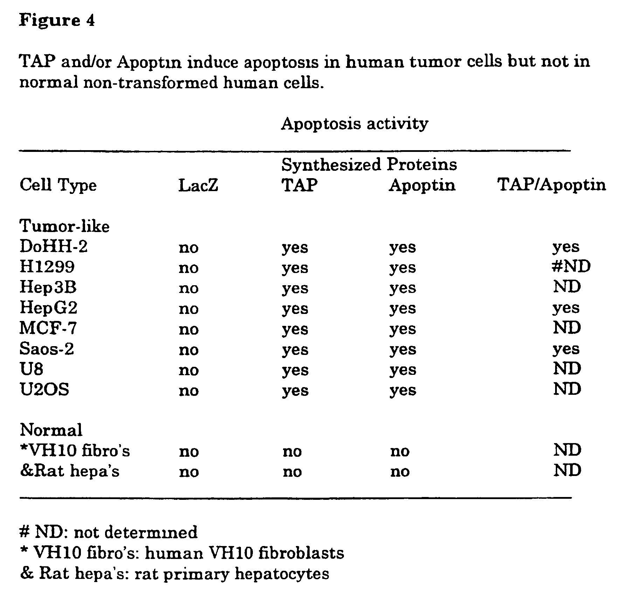

Surprisingly, such a protein according to the invention is capable of providing apoptosis alone or in combination with another apoptosis-inducing substance, as for example shown in Figure 4, in particular wherein said apoptosis-inducing substance comprises Apoptin or a functional fragment thereof. In a particular advantageous embodiment of the invention, said protein according to the invention is capable of providing apoptosis independent of p53 and/or does not need a functional caspase-3.

Preferable the protein according to the invention provides tumor-specific apoptosis and does not or at least not significantly induce apoptosis in normal cells. Even more preferably, a protein according to the invention, or fragment thereof, comprises a nuclear location signal and/or a nuclear export signal as for example shown in Figure 2.

Protein, herein also called TAP protein, is defined as a substance comprising a peptide, optionally having been modified by for example glycosylation, myristilation, phosphorylation, the addition of lipids, by homologous or heterologous di- or multimerisation, or any other (posttranslational) modifications known in the art.

TTV-derived protein herein is defined as a protein for example encoded by strains of the recently discovered novel human single-stranded circular DNA virus TTV (Charlton et al., 1998; Miyata et al., 1999). Thus far, TTV and TTV-like viruses have not been correlated to any known disease. However, TTV contains also a single-stranded circular DNA which relates it to CAV, from which Apoptin is derived. TTV has a similar genomic organization like CAV, however, none of the predicted open reading frames of TTV were proven to be transcribed and translated in TTV-infected cells (Miyata et al., 1999). In particular, the herein described TTV open reading frame has not been shown to be transcribed and translated *in vivo*, and no such TTV protein has until now been identified, let alone that a function has been assigned to it, at least not a function related to apoptotic activity.

A functional equivalent and/or a functional fragment thereof is a derivative and/or a fragment having the same kind of activity (capable of providing apoptosis) possibly in different amounts.In a preferred embodiment, the invention provides an isolated or recombinant nucleic acid or functional equivalent and/or functional fragment thereof encoding and/or capable of expression of a TTV-derived protein capable of providing apoptosis. In particular, the invention provides an isolated or recombinant nucleic acid or functional equivalent and/or functional fragment thereof from which a protein according to the invention can be transcribed and translated.

In another embodiment, the invention provides an isolated or recombinant nucleic acid or functional equivalent and/or fragment thereof encoding a TTV-derived protein capable of providing apoptosis capable of hybridizing to a nucleic acid molecule encoding a TTV-derived protein capable of providing apoptosis as shown in Figure 1, in particular encoding a novel protein capable of providing apoptosis or functional equivalent and/or functional fragment thereof called TTV-apoptosis protein, abbreviated as TAP.

In particular, the invention provides an isolated or recombinant nucleic acid or fragment thereof encoding, and preferably capable of expressing a TAP substance capable of providing apoptosis being at least 60% homologous, preferably at least 70%, more preferably at least 80%, even more preferably at least 90%, most preferably at least 95% homologous to a nucleic acid molecule, or to a functional fragment thereof, encoding a TAP protein as shown in Figure 1.

Furthermore, the invention provides a vector comprising a nucleic acid encoding a protein according to the invention. Examples of such a vector are given in the detailed description given herein; such as vector pCMV-TAP, a plasmid expressing a TAP product, and so on.

In yet another embodiment, the invention provides a vector comprising a nucleic acid encoding a protein according to the invention, said vector comprising a gene-delivery vehicle, making the invention very useful in gene therapy. By equipping a gene delivery vehicle with a nucleic acid according to the invention, and by targeting said vehicle to a cell or cells that behave over-proliferating and/or have shown decreased death rates, said gene delivery vehicle provides said cell or cells with the necessary means of apoptosis, providing far reaching therapeutic possibilities.

Furthermore, the invention provides a transformed host cell comprising a (recombinant) nucleic acid encoding a protein or a vector according to the invention. Examples comprise transformed or transfected bacterial cells as described in the detailed description herein. Preferred is a host cell according the invention which is a transformed eukaryotic cell, such as mammalian cells transformed or transfected with a nucleic acid or vector according to the invention. Said cells are in general capable to express or to produce a protein capable of providing apoptosis.

The invention furthermore provides an isolated or recombinant TAP protein capable of inducing apoptosis. Expression of such TAP substances in cells, induces the apoptotic process. It can do so alone, or in the presence of other apoptosis inducing substances such as Apoptin, and especially so independent of p53 and/or insensitive to anti-apoptosis proteins such as Bcl-2 and/or in cases wherein functional caspase-3 is lacking, showing that also in those cases where (functional) p53 is absent and/or active Bcl-2 is present and/or functional caspase-3 is lacking apoptosis can be induced by a substance according to the invention.

In particular, the invention provides a protein according to the invention encoded by a nucleic acid according to the invention, for example comprising at least a part of an amino acid sequence as shown in Figure 2 or a functional fragment thereof capable of providing apoptosis alone or in combination with other apoptosis inducing substances such as Apoptin.

The invention also provides an isolated or synthetic antibody specifically recognizing a protein or functional fragment thereof according to the invention. Such an antibody is for example obtainable by immunizing an experimental animal with a TAP proteinaceous substance or an immunogenic fragment and/or equivalent thereof and harvesting polyclonal antibodies from said immunized animal, or obtainable by other methods known in the art such as by producing monoclonal antibodies, or (single chain) antibodies or binding proteins expressed from recombinant nucleic acid derived from a nucleic acid library, for example obtainable via phage display techniques.

With such an antibody, the invention also provides a protein specifically recognizable by such an antibody according to the invention, for example obtainable via immunoprecipitation, Western Blotting, or other immunological techniques known in the art.

Furthermore, the invention provides use of a nucleic acid, vector, host cell, or protein according to the invention for the induction of apoptosis. In particular, such use is also provided further comprising use of a nucleic acid encoding Apoptin or a functional equivalent and/or functional fragment thereof or use of Apoptin or a functional equivalent and/or functional fragment thereof. Combining these apoptosis-inducing substances increases the percentage of apoptosis in treated tumor cells (see for example Figure 4). In particular, such use is provided wherein said apoptosis is p53-independent and/or Bcl-2 insensitive and/or caspase-3 independent.

Such use as provided by the invention is particularly useful from a therapeutic viewpoint. The invention provides herewith a pharmaceutical composition comprising a nucleic acid, vector, host cell, or protein according to the invention. In addition, such a pharmaceutical composition according to the invention is provided further comprising a nucleic acid encoding Apoptin or a functional equivalent or fragment thereof or Apoptin or a functional equivalent or fragment thereof.

Such a pharmaceutical composition is in particular provided for the induction of apoptosis, for example wherein said apoptosis is p53-independent and/or Bcl-2 insensitive and/or caspase-3 independent, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. Also, use of a nucleic acid, vector, host cell, or protein according to the invention is provided for the preparation of a pharmaceutical composition for the treatment of a disease where enhanced cell proliferation or decreased cell death is observed, as is in general the case when said disease comprises cancer or auto-immune disease.

The invention further discloses the apoptotic activity of a protein according to the invention (also called TTV-apoptosis inducing protein or TAP; the terms may be used interchangeable herein) in tumor versus normal cells; said protein fails to induce apoptosis in normal fibroblast and hepatocytes and can therefor be used for depletion of specifically tumor cells. So TAP, just like Apoptin, is a very potent anti-tumor agent. Expression of TAP can be used for the induction of apoptosis in specifically (human) tumor cells. TAP, again just like Apoptin, does not or at least not significantly induce apoptosis in normal cells, indicating that the toxicity of TAP-treatment will be low.

TAP can be (transiently) expressed in tumors by means of DNA transfection. Expression of TAP in (tumor) cells may take place by infecting cells with (retro) viral vectors that contain a coding sequence for a protein according to the invention, capable of providing apoptosis, alone or in combination with other apoptosis-inducing substances, such as Apoptin. Administration to cells of non-viral components (e.g. liposomes or transferrin-derived vectors) containing a protein according to the invention and/or a nucleic acid according to the invention is a further possibility for the expression/presence of a protein according to the invention and induction of apoptosis in tumor cells. Such a non-viral component according to the invention may further comprise Apoptin and/or coding sequences for Apoptin.

Furthermore, we have provided evidence that TAP, like Apoptin, does not need functional p53, cannot be blocked by inhibitors of the p53 apoptotic pathway, as for example the Bcl-2 proteins and does not need functional caspase-3. Together with the fact that TAP induces apoptosis in different tumor cell, as disclosed herein, all these data imply that TAP can induce apoptosis in different tumor cells and is a very potent inducer of apoptosis in tumor cells, which can overcome blocks of anti-apoptotic activity in tumor cells. Therefore, TAP is a potent anti-tumor agent for a broad variety of tumor(s) (cells). gene encoding a tumoricidal substance mainly, but not exclusively to a tumor cell, characterised in that the tumoricidal substance is TAP. In addition, such a vehicle according to the invention may further comprise Apoptin or a nucleic acid encoding Apoptin or a functional fragment thereof. In the prior art many vehicles for delivering cytotoxic agents or precursors thereof have been disclosed. A major problem in delivering cytotoxic agents is that they are harmful to all cells and not just the tumor cells. Therefore many different ways of targeting the cytotoxic substance to the tumor cells have been investigated. Although many targeting moieties are nowadays known, essentially all of them suffer from the drawback of not being completely specific for the tumor target. Therefore the use of this cytotoxic substance has so far not met with great success. Experiments as disclosed herein for TAP have shown that this is not the case for TAP and as already disclosed before this is also the case for Apoptin. TAP and/or Apoptm only have a significant effect in tumor cells and not in normal cells, at least not significantly. Thus, even if the targeting moiety or any other means of delivery of TAP and/or Apoptin is not very specific, this will result in hardly any toxicity to normal tissue. Thus the invention provides a conjugate for targeted tumor therapy comprising a targeting moiety having binding affinity for a molecule associated mainly, but not exclusively with the surface of a tumor cell and further comprising TAP. In addition such a conjugate according to the invention may further comprise Apoptm.

A targeting moiety is well defined in the art as a molecule with a specific binding activity for a target molecule. It should preferably be capable of mternalisation. The target molecule may be an antigen or an epitope, in which the targeting moiety is an antibody or a fragment or a derivative thereof. The target molecule may be a receptor in which case the targeting moiety is a ligand for said receptor (for example EGF and its receptor). These are just examples of suitable combinations. The bond between the targeting moiety and TAP has only one requirement in that it should allow the functions of both partners to operate. The same requirement is applicable for Apoptin if the invention further comprises Apoptin. Thus, it may be a chemical (labile) bond, it may be a fusion protein. The conjugate may even be a liposome covered with targeting moieties and filled with TAP and optionally Apoptin (or gene(s) encoding such a protein). The invention also comprises a vehicle delivering a gene encoding TAP to a tumor cell, using gene therapy. Such a vehicle according to the invention may also further comprise Apoptin or its encoding gene. Gene therapy has many well known methods to deliver genes to cells using viruses such as adenovirus or retrovirus. The person skilled in the art will know how to select the right vehicle. TAP and Apoptin only functions to induce apoptosis in transformed cells, it or its and/or they or theirs gene(s) can be used as a safety measure in other gene therapy regimens than tumor therapy, such as those rectifying deficiencies from mheritable diseases. It is then provided in a gene delivery vehicle together with the gene of interest and if the cell in which the gene of interest becomes malignant then it will be susceptible to the action of TAP. Thus the invention provides a vehicle for delivering a nucleic acid of interest to a target cell, said vehicle further comprising a nucleic acid according to the invention. Such a vehicle according to the invention may further comprise Apoptm.

The invention further provides TAP for use in a method of eliminating cells of a target cell population whereby the method is mainly, but not entirely specific for the cells of the target population, whereby TAP is the cytotoxic agent, as well as TAP for use in a method of eliminating cells of a target cells of a target cell population whereby the cells of said population are not sensitive to other apoptosis inducing agents.

The latter is possible because TAP and Apoptm cannot be inhibited by the mechanism inhibiting the other apoptosis inducing substances.

The last two mentioned methods according to the invention may further comprise Apoptm

The expressions "mainly but not exclusively" and "mainly but not entirely" reflect the finding that TAP (and optionally Apoptin) is/are only active in tumor cells and not, at least not significantly in normal cells and it is therefor no longer necessary to provide a targeting means which is exclusively found on tumor cells.

The invention will be explained m more detail in the following detailed description, which is not limiting the invention.

### Detailed description

The invention provides a method for inducing apoptosis through interference with the function of this newly discovered apoptosis-related TAP protein or functional equivalents or fragments thereof and/or the induction of apoptosis by means of (over)expression of TAP or related gene or functional equivalents thereof.

The invention also provides an anti-tumor therapy based on the interference with the function of TAP-like proteins and/or its (over)expression. The invention provides a therapy for cancer, auto-immune diseases or related diseases which is based on TAP-like proteins alone or in combination with Apoptm and/or Apoptin-like compounds.

### DNA cloning

The various cloning steps have been carried out according to the methods described by Mamatis et al., 1982.

For drug selections Luria Broth (LB) plates for E.coli were supplemented with ampicillin (50 microgram per ml).

### Sequence analysis

The clones containing the sequences encoding TAP proteins were sequenced using dideoxy NTPs according to the Sanger-method, which was performed by BaseClear, Leiden, The Netherlands. The used sequencing primer was a 20-mer of the DNA-sequence 5'-ggatccctagcaggtctgcg-3'. Primary rat hepatocytes

Primary rat hepatocytes were isolated from collagenase-perfused livers as standard and cultured in Williams E medium (Gibco Life Technologies, Grand Island, NY, USA) supplemented with insulin (2 mU/ml) and dexamethasone (1mM). The cells were grown on collagen-coated culture slides (Micronic, Lelystad, The Netherlands).

### Transfection methods

Plasmid DNA was purified by QIAGEN-column chromatography according to the instructions of the manufacturer (Westburg BV, Leusden, The Netherlands).

The various mammalian cell lines were grown in the appropriate culture medium supplemented with penicillin and streptomycin (GIBCO/BRL).

The cells were transfected by calcium-phosphate precipitation as described (Graham and Van der Eb, 1973) or transfected with Fugene, according the instructions provided by the manufacturer (Roche, Almere, The Netherlands).

### Micro-injection of plasmid DNA encoding TAP and control genes.

Cells were micro-injected in the cytoplasm with plasmid pCMV-TAP (cloning as described herein), pCMV-LacZ or pCMV-Apoptin (Danen-Van Oorschot et al., 1997) at a concentration of 50 ng per ul using an Eppendorf micro-injector with the injection pressure of 0.5 psi. The cells were co-injected with Dextran-rhodamine (MW: 70 kDa; Molecular probes, Leiden , The Netherlands) to be able to later identify injected cells. The cells were incubated at 37°C after injection until the cells were fixed.

### Immunofluorescence assays

Cells were fixed with 80% acetone and used for immunofluorescence tests with myc-tagged- and/or Apoptin-specific monoclonal antibodies with fluorescein-isothiocyanate (Noteborn et al., 1998)

### Apoptosis assays

The apoptotic cells were initially determined by means of DAPI staining (Telford et al., 1992). DAPI stains intact cellular DNA strongly and regularly, whereas it stains apoptotic DNA weakly and/or irregularly.

The TUNEL assay was carried out as described by Pietersen et al., 1999 with the use of an m-situ cell death detection kit (Roche, Almere, The Netherlands).

### Western-blot analysis

The cells were harvested after transfection and lysed in 2 x sample buffer [100 mM TrisHCl (pH 6.8), 4% SDS, 20% glycerol, 0.2% bromophenol blue, 2.5 % beta-mercaptoethanol]. Proteins were fractionated on 15% SDS-polyacrylamide gels and electroblotted onto Immobilon-P membranes (Millipore). Parts of the blots were then incubated with Mab 9E10 (1:5000) to myc-tag to detect TAP. Positive signals were visualized by enhanced chemiluminescence according to the manufacture's protocol (Amersham, The Netherlands).

### Results and discussion

### Construction of a plasmid containing the TAP gene.

To examine whether TAP protein harbors apoptotic activity we have to make a DNA construct containing the complete TAP DNA sequences, which are shown in Figure 1. In addition, the stop codon has been also indicated. The presented TAP DNA sequence synthesizes a putative protein of 105 amino acids (Figure 2). Furthermore, at the N-terminal end of the TAP gene, a Myc-tag (amino acids: EQKLISEEDL) has been linked which is in frame with the TAP gene product. This Myc-tag enables the recognition of the TAP proteins by means of the Myc-tag-specific 9E10 antibody.

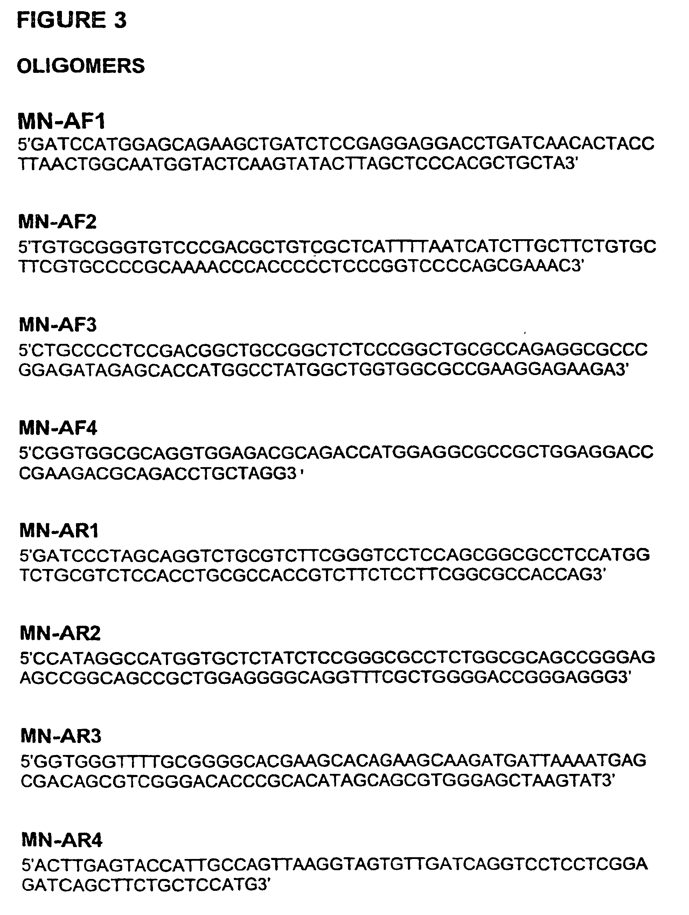

The DNA construct containing the TAP DNA sequences, Myc-tag DNA sequences and *Bam*H1 restriction-enzyme sites has been made by using a panel of DNA oligomers as shown in Figure 3. All steps for making the required approximately 0.36 kb DNA fragment was carried by The Midland Certified Reagent Co., Midland, Texas, USA.

In principle the synthesized DNA oligomers were synthesized and purified by preparative polyacrylamide gelelectrophoresis and 5'-phosphorylated to permit ligation. Subsequently, the various oligomers were annealed and then ligated en masse in a thermostable ligation reaction. The long internal overlaps annealed stably and are efficiently ligated, whereas the cohesive termini tend to remain dissociated. As a consequence, the ligation product could be directly purified by preparative low-melting-point agarose gel electrophoresis using the freeze-squeeze method. The appropriated approximately 0.36 kb DNA fragment was made visible in the gel with long wavelength UV light. Next, the DNA band was excised and purified and ligated in *Bam*H1-linearized pUC19 plasmid DNA, which was treated with calf intestine alkaline phosphatase. The final product was analyzed by *Bam*H1 digestion for containing an approximately 0.36 kb DNA fragment and sequence analysis. The plasmid DNA containing the myc-tagged TAP DNA sequences has been called pMN-TAP.

### Construction of an expression vector for the identification of TAP protein in human and other mammalian cells.

To study whether the cloned myc-tagged TAP gene encode protein products, we have carried out the following experiments. The DNA plasmid pcDNA3.1 (In Vitrogen, Groningen, The Netherlands) contains the cytomegalovirus (CMV) promoter enabling high levels of expression of foreign genes in transformed human and other mammalian cells, such as Saos-2 and Cos cells.

The pcDNA3.1 construct expressing Myc-tagged TAP DNA was constructed as follows. The pMN-TAP clone was digested with the restriction enzyme *Bam*H1 and the requested approximately 0.36 kb DNA insert was isolated. The expression vector pcDNA3.1 digested with *Bam*H1 and treated with calf intestine alkaline phosphatase and ligated to the isolated myc-tagged TAP DNA insert. By restriction-enzyme digestions and sequence analysis, the pcDNA3.1 construct containing the myc-tagged TAP DNA in the correct orientation was identified. The final construct containing the TAP sequences is called pCMV-TAP.

The synthesis of the Myc-tagged TAP protein was analyzed by transfection of Cos cells with plasmid pCMV-TAP. As negative control, Cos cells were mock-transfected. Two days after transfection, the cells were lysed and Western-blot analysis was carried out using the Myc-tag-specific antibody 9E10.

The Cos cells transfected with pCMV-TAP were proven to synthesize a specific Myc-tagged TAP product with the expected size of approximately 12 kDa. As expected, the lysates of the mock-transfected Cos cells did not contain a protein product reacting with the Myc-tag-specific antibodies.

These results indicate that we have been able to construct a plasmid that is able to produce a protein product derived from the putative TAP open reading frame.

### Over-expression of the novel TAP protein in human hepatoma HepG2 cells induces the apoptotic process.

Next, we have examined whether TAP carries apoptotic activity. First, we have analyzed the cellular localization of the novel TAP protein in human hepatoma-derived HepG2 cells, which are synthesizing functional tumor suppressor protein p53 (Pietersen et al., 1999). To that end, the HepG2 cells were transfected with plasmid pCMV-TAP encoding the myc-tagged TAP protein, as described by Danen-van Oorschot (1997). As a negative control, HepG2 cells were transfected with a plasmid encoding myc-tagged lacZ.

Two days after transfection, the cells were fixed. By indirect immunofluorescence using the myc-tag-specific antibodies 9E10 and DAPI, which stains the nuclear DNA, it was shown that the TAP protein was present in the cytoplasm of the cell.

Finally, we examined whether (over)-expression of our novel TAP protein results in induction of apoptosis. To that end, we have analyzed the TAP-positive cells for DAPI-staining. Four days after transfection, almost all TAP-positive cells were aberrantly stained with DAPI, which is indicative for induction of apoptosis (Telford, 1992, Danen-van Oorschot, 1997). At this time point, TAP was localized in the nuclear structures of the transfected HepG2 cells. The nuclear DNA of the cells expressing lacZ was not aberrantly stained with DAPI. Therefore, the obtained results (Figure 4) indicate that TAP can induce apoptosis in human tumor cells.

### Over-expression of the novel TAP protein in human osteosarcoma U2OS cells expressing wild-type p53 versus human osteosarcoma Saos-2 and hepatoma Hep3B cells lacking functional p53.

During tumor development, most of the tumors will lack functional tumor suppressor p53. Tumor cells lacking functional p53 are in general poor responders to (chemo)therapeutic agents. In the above described experiment, we have shown that TAP can induce apoptosis in cells containing functional tumor suppressor protein p53. Therefore, it is also of importance to prove whether TAP can induce apoptosis in human tumor cells in the absence of functional p53.

To that end, we have transfected human osteosarcoma-derived Saos-2 cells and human hepatoma-derived Hep3B cells synthesizing no tumor suppressor p53 (Zhuang et al. 1995a, 1995b) with plasmid pCMV-TAP encoding myc-tagged TAP protein, U2OS cells expressing wild-type p53 were used as a control in this experiment. As control, the three different cell lines were transfected with the plasmid pCMV-LacZ encoding myc-tagged LacZ. Four or five days after transfection, the cells were fixed and analysed for myc-tagged TAP expression by means of immunofluorescence using the myc-tag specific antibody 9E10 and apoptotic activity by DAPI staining. In parallel, transfected Saos-2, U2OS and Hep3B cells were fixed and examined for induction of apoptosis by means of a TUNEL assay (Pietersen et al., 1999). An equal significant amount of Saos-2 and U2OS cells were stained aberrantly with DAPI whereas the cells containing myc-tagged LacZ were not, which is indicative for induction of TAP-specific apoptosis to a similar level in p53-minus Saos-2 versus p53-positive U2OS cells. In addition, Saos-2 and U2OS cell cultures transfected with pCMV-TAP showed a significant higher amount of TUNEL-positive cells than Saos-2 cell cultures transfected with pCMV-LacZ.

Furthermore, the TAP-positive Hep3B cells underwent also apoptosis as assayed both with the DAPI-staining method as well as with the TUNEL assay. The level of apoptosis induction was similar to the level of apoptosis found induction in HepG2 cells.

The results (Figure 4), obtained with TAP synthesis in both HepG2 versus Hep3B and Saos-2 versus U20S cells, prove that TAP can induce apoptosis independent of p53. Therefore, tumors or tumorous cells having functional p53 or not can be treated with TAP-synthesizing vectors.

### Over-expression of the novel TAP protein in human lymphoma DoHH-2 cells induces the apoptotic process.

Besides non-functional p53, over expression of anti-apoptosis proteins such as Bcl-2 hamper conventional anti-cancer therapies. Therefore, we have also examined whether TAP can induce apoptosis in human tumor cells over-expressing Bcl-2 such as DoHH-2 cells (Zhuang et al., 1995). To that end, DoHH-2 cells were transfected with pCMV-TAP or pCMV-lacZ and analyzed as described for the HepG2 cells. Only the DoHH-2 cells synthesizing TAP protein were shown to be stained aberrantly with DAPI.

Therefore, we conclude that TAP also induces apoptosis in cells over-expressing Bcl-2. The results are shown in Figure 4.

### Co-expression of TAP and Apoptin induces apoptosis in human tumor cells in a very efficient way.

The CAV-derived protein Apoptin induces apoptosis upon nuclear localization as seems also to be the case for TAP-induced apoptosis. However, the TAP protein is present m a more diffused way in the cytoplasm in comparison to Apoptin. Furthermore, TAP seems to enter the nucleus shortly before the cells undergo apoptosis, whereas Apoptin does earlier (see above and below). In the following transfection experiments (carried out as described above), we have examined the rate of apoptosis induction m human tumor cells synthesizing both TAP and Apoptin m comparison with tumor cells synthesizing only TAP or Apoptm.

All 3 examined human tumor cell lines HepG2, Saos-2 and DoHH-2 underwent a significant higher level of apoptosis after co-expression of Apoptin and TAP proteins m comparison with the cells expressing Apoptin or TAP separately. The results are shown in Figure 4.

### TAP induces apoptosis in cells lacking essential factors of the caspase-regulated apoptosis execution pathway.

Essential factors in the apoptotic machinery are the specific proteases called caspases (Danen-Van Oorschot et al., 2000 and references herein). Apoptm was shown to be able to induce apoptosis in human breast-tumor derived MCF-7 cells lacking the essential execution caspase-3 (Danen-Van Oorschot et al., 2000).

To test if this is also the case for TAP, we have transfected human MCF-7 cells with plasmid pCMV-TAP encoding myc-tagged TAP protein. As control, the cells were transfected with the plasmid pCMV-LacZ encoding the myc-tagged LacZ, as was the case for all above described experiments. Four or five days after transfection, the cells were fixed and analysed for myc-tagged TAP expression by means of immunofluorescence using the myc-tag specific antibody 9E10 and apoptotic activity by DAPI staining.

A significant amount of MCF-7 cells were stained aberrantly with DAPI whereas the cells containing LacZ were not, which is indicative for induction of TAP-specific apoptosis. The level of apoptosis was to a comparable level as in p53-minus Saos-2 cells or p53-positive U2OS cells, which indicates that despite the fact that functional caspase-3 is absent in these human tumor cells, TAP is able to force these cells undergoing apoptosis.

### TAP induces apoptosis in cell lines derived from human lung-tumor and squamous cell carcinomas.

Next, we have examined whether cell lines derived from human lung tumors and squamous cell carcinoma were sensitive to TAP-induced apoptosis.

To that end, we have transfected human lung-tumor H1299 cells (Friedlander et al., 1996) and human squamous cell carcinoma U8 cells (van Damme et al., 1997) with plasmid pCMV-TAP encoding myc-tagged TAP protein. As control, the cells were transfected with the plasmid pCMV-LacZ expressing the myc-tagged LacZ. Four or five days after transfection, the cells were fixed and analysed for myc-tagged TAP expression by means of immunofluorescence using the myc-tag specific antibody 9E10 and apoptotic activity by DAPI staining.

A significant amount of H1299 and U8 cells were stained aberrantly with DAPI whereas the cells containing LacZ were not, which is indicative for induction of TAP-specific apoptosis.

In conclusion, we have provided evidence that expression of TAP proteins in various human tumorigenic cells results in induction of apoptosis. Therapies based on induction of (p53-independent and/or Bcl-2 insensitive and/or caspase-3 independent) apoptosis are possible utilizing the TAP proteins. Combined therapies can be carried out using TAP protein and other apoptosis-inducing proteins such as Apoptin. Another CAV-derived protein, which is known to induce apoptosis and also known to enhance Apoptin activity is VP2 (Noteborn et al., 1997).

### TAP induces apoptosis in a tumor-specific way.

An efficient anti-tumor therapy without obvious side effects is by far the preferable one. Therefore, we have examined whether TAP can induce apoptosis in normal human fibroblasts. In addition, we have also analysed the TAP apoptosis activity in rat primary hepatocytes.

To that end, we have transfected human VH10 fibroblasts (Danen-Van Oorschot et al. 1997) with plasmid pCMV-TAP encoding myc-tagged TAP protein. As control, the cells were transfected with the plasmid pCMV-LacZ encoding myc-tagged LacZ or with pCMV-Apoptin encoding Apoptin (negative controls). Four or five days after transfection, the cells were fixed as described above. In parallel, human transformed NW18 fibroblasts (Danen-Van Oorschot et al., 1997) were examined for TAP-induced apoptosis as described for the human normal fibroblasts

By means of micro-injection of rat primary hepatocytes with plasmids pCMV-TAP, pCMV-Apoptin or pCMV-LacZ, we examined whether TAP was able to induce apoptosis in these normal rat hepatocytes. In parallel, human transformed HepG2 cells were micro-injected with the above described plasmids encoding myc-tagged TAP, Apoptin or myc-tagged LacZ protein, respectively. Six and 24 hours after micro-injection the cells were fixed.

The cells were analysed for myc-tagged TAP or myc-tagged LacZ expression by means of immunofluorescence using the myc-tag specific antibody 9E10 or the Apoptin expression by using the Apoptin-specific antibody 111.3 and apoptotic activity by DAPI staining.

The human VH10 fibroblasts and rat hepatocytes containing TAP protein were stained aberrantly with DAPI to a background level as the cells containing LacZ or Apoptin, whereas the transformed/tumorigenic controls samples NW18 and HepG2 cells clearly showed high levels of apoptosis induction when synthesizing TAP or Apoptin but not when produce/contain LacZ. This data are indicating that TAP does not or at least not significantly induce apoptosis in the human and rodent normal cells.

Therefore, we conclude that TAP like Apoptm induces tumor-specifically apoptosis.

### Production of polyclonal antibodies directed against TAP proteins.

For the production of polyclonal antibodies against TAP proteins three putative immunogenic peptides were synthesized:
N-terminus-THPLPVPSETCPSDG-C-terminus;
N-terminus-CRLSRLRQRRPEIEH-C-terminus;
N-terminus-CTQTMEAPLEDPKTQT-C-terminus;
(EuroGentec SA, Seraing, Belgium).

Subsequently, co-workers of EuroGentec injected two rabbits with the TAP-specific peptides according to standard procedures.

The serum derived from the rabbits injected with the TAP peptides was shown to recognize specifically for in this report described TAP products by means of immunofluorescence and Western-blot assays. These results imply that we have generated specific antibodies, which can be used for detecting our novel TAP protein. Furthermore, this antibody forms the basis for a diagnostic test in detecting TAP in sera of TTV-infected individuals, which is indicative for a TTV strain with apoptotic activity.

### Functional domains of TAP

Although, TAP does not share overall sequence similarities with any known protein sequence and m particular with the CAV-derived apoptosis-inducing protein Apoptin, it also has apoptotic activity m human tumor cells. TAP harbors putative sequences resembling a nuclear location signal or a nuclear export signal as indicated m Figure 2. Furthermore, TAP has a relative high percentage (>8%) of proline residues. Computer analysis revealed a significant structural homology among TAP and Apoptin, which might explain the similarity in their activity.

### Description of the figures

Figure 1 shows the DNA sequence encoding TAP protein which has apoptotic activity in (human) tumor cells.
Figure 2 shows an ammo-acid sequence of TAP protein.
   The NES domain is located on the TAP protein in between ammo acids 27 and 39. The NLS domain is situated in the TAP protein from position 62 and 68.
Figure 3 shows the scheme of the used oligomers to construct pMN-TAP. Besides the TAP sequence the oligomers contain a myc-tag and BamH1 restriction-enzyme site, which are required for binding to the myc-tag-specific antibody 9E10 and enabling cloning in a BamH1 restriction-enzyme site, respectively.
Figure 4 shows the apoptotic activity of TAP protein and/or Apoptin in various human tumor cell lines.
   DoHH-2: human lymphoma-derived cell line over-expressing Bcl-2; HepG2: human hepatoma-derived cell line expressing wild-type p53; Saos-2: human osteosarcoma-derived cells lacking functional p53; H1299: human lung carcinoma cells; Hep3B:human hepatoma cells lacking functional p53; MCF-7:human breast tumor cells lacking caspase-3 activity;U8:human squamous cell carcinoma cells lacking functional p53, U2OS:human osteosarcoma cells which are functionally compotent for p53 function.

### References

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Charlton, M., Adjei, P., Poterucha, J., Zein, N., Moore, B., Therneau T., Krom, R., and Wiesner, R. 1998. TT-virus infection in North American blood donors, patients with fulminant hepatic failure, and cryptogenic cirrhosis. Hepatology 28, 839-842.
3. Van Damme, H., Brok, H., Schuunng-Scholtes, E., and Schuuring, E (1997). The redistribution of cortactin into cell-matrix contact sites in human carcinoma cells with 11q13 amplification is associated with both overexpression and post-translational modification. Journal Biological Chemistry 272: 7374-7380.
4. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
5. Danen-Van Oorschot, A.A.A.M, Den Hollander, A., Takayama, S., Reed, J., Van der Eb, A.J. and Noteborn, M.H.M. (1997a). BAG-1 inhibits p53-induced but not Apoptin-induced apoptosis. Apoptosis 2, 395-402.
6. Danen-Van Oorschot, A.A.A.M., Van der Eb, A.J., and Noteborn, M.H.M. (2000). The chicken anemia virus-derived protein Apoptm requires activation of caspases for induction of apoptosis in human tumor cells. Journal of Virology 74, 7072-7078.
7.. Duke, R.C., Ocjius, D.M., Young, J, D-E. (1996). Cell suicide in health and disease. Scientific American December 1996, 48-55.
8. Earnshaw, W.C., 1995 Nuclear changes in apoptosis. Current Opinion in Cell Biology 7, 337-343.
9. Friedlander, P., Haupt, Y., Prives, C., and Oren, M. (1996). A mutant p53 that discriminates between p53-responsive genes cannot induce apoptosis. Molecular Cell Biology 16: 4961-4971.
10. Hockenberry, D.M. (1994). Bcl-2 in cancer, development and apoptosis. Journal of Cell Science, Supplement 18, 51-55.
11. Kerr, J.F.R., Winterford, C.M., and Harmon, B.V. (1994). Apoptosis: Its significance in cancer and cancer therapy. Cancer 73, 2013-2026.
12. Levine, A.J. (1997). p53, the cellular gatekeeper for growth and division. Cell 88, 323-331.
13. Maniatis, T., Fritsch, E.F., and Sambrook, J. (1982).Molecular Cloning: A Laboratory Manual. CSHL Press, New York, USA.
14. McDonell T.J., Meyn, R.E., Robertson, L.E. (1995). Implications of apoptotic cell death regulation in cancer therapy. Seminars in Cancer Biology 6, 53-60.
15. Miyata, H., Tsunoda, H., Kazi, A. Yamada, A., Khan, M.A., Murakami, J., Kamahora, T., Shiraki, K., and Hino, S. (1999). Indentification of a novel GC-rich 113-nucleotide region to complete the circular, single-stranded DNA genome of TT virus, the first human circovirus. Journal of Virology 73, 3582-3586.
16. Noteborn, M.H.M. (1996). PCT application WO 96/41191. Apoptin induces apoptosis in human transformed and malignant cells but not in normal cells as essential characteristic for the development of an anti-tumor therapy.
17. Noteborn, M.H.M., and De Boer, G.F. (1996). Patent USA/no. 030, 335.
18. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
19. Noteborn, M.H.M., and Pietersen, A. (1998). A gene delivery vehicle expressing the apoptosis-inducmg proteins VP2 and/or Apoptin. PCT Application no. NL98/00213.
20. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
21. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology. 79, 3073-3077.
22. Noteborn, M.H.M., and Zhang, Y. (1998). Methods and means for determining the transforming capability of agents, for determining the predisposition of cells to become transformed and prophylactic treatment of cancer using Apoptin-like activity. PCT Application NL98/00457.
23. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
24. Paulovich, A.G., Toczyski, D., Hartwell, H. (1997). When checkpoints fail. Cell 88, 315-321.
25. Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the Apoptin gene. Gene Therapy 6, 882-892.
26. Rose, M.D., Winston, F., and Hieter, P. (1990). Methods in yeast genetics. A laboratory course manual. Cold Spring Harbor Laboratory Press, Cold Spring Harbor, New York, USA.
27. Sachs, L. and Lotem, J. (1993). Control of programmed cell death in normal and leukemia cells: New implications for therapy. Blood 82, 15-21.
28. Sanger, F., Nicklen, S., and Coulsen, A.R. (1977). DNA sequencing with chain-terminating inhibitors. Proceedings National Academic Sciences USA 74, 5463-5467.
29. Steller, H. (1995). Mechanisms and genes of cellular suicide. Science 267, 1445-1449.
30. Telford, W.G., King, L.E., Fraker, P.J. (1992). Comparative evaluation of several DNA binding dyes in the detection of apoptosis-associated chromatin degradation by flow cytometry. Cytometry 13, 137-143.
31. Teodoro, J.G. and Branton, P.E. (1997). Regulation of apoptosis by viral gene products. Journal of Virology 71, 1739-1746.
32. Thompson, C.B. (1995). Apoptosis in the pathogenesis and treatment of disease. Science 267, 1456-1462.
33. White, E. (1996). Life, death, and the pursuit of apoptosis. Genes and development 10, 1-15.
34. Wyllie, A.H. (1995). The genetic regulation of apoptosis. Current Opinion in Genetics and Development 5, 97-104.
35. Wyllie, A.H., Kerr, J.F.R., Currie, A.R. (1980). Cell death: The significance of apoptosis. International Review of Cytology 68, 251-306.
36. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells *in vitro*. Leukemia 9 S1, 118-120.
37. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.
38. Zhuang, S.-M., Shvarts, A., Van Ormondt, H., Jochemsen, A.-G., Van der Eb, A.J., and Noteborn, M.H.M. (1995b). Differential sensitivity to Ad5 E1B-21kD and Bcl-2 proteins of Apoptin-induced versus p53-induced apoptosis. Carcinogenesis 16: 2939-2944.

## Claims

1. Apoptosis inducing protein, wherein said protein comprises the amino acid sequence as shown in Figure 2 or a functional fragment thereof, wherein said fragment has the function of amino acid sequence as shown in Figure 2.

2. Protein according to claim 1 capable of providing apoptosis further in combination with another apoptosis-inducing substance.

3. Protein according to claim 2 wherein said apoptosis-inducing substance comprises Apoptin or a functional fragment thereof.

4. Protein according to anyone of claims 1 to 3 capable of providing apoptosis independent of p53.

5. Protein according to anyone of claims 1 to 4 capable of providing tumor specific apoptosis.

6. Protein according to anyone of claims 1 to 5 capable of providing apoptosis independent of caspase-3.

7. Protein according to anyone of claims 1 to 6 comprising a nuclear location signal and/or a nuclear export signal.

8. An isolated or recombinant nucleic acid or functional fragment thereof from which a protein according to anyone of claims 1 to 7 can be transcribed and translated.

9. A vector comprising a nucleic acid according to claim 8.

10. A vector according to claim 9 comprising a gene-delivery vehicle.

11. A host cell comprising a nucleic acid according to claim 8 bearing a vector according to claim 9 or 10.

12. A host cell according to claim 11 which is a eukaryotic cell such as a yeast cell or a vertebrate cell.

13. An isolated or synthetic antibody specifically recognising a protein or functional fragment, thereof according to anyone of claims 1 to 7.

14. A protein specifically recognisable by an antibody according to claim 13, wherein said protein has the function of amino acid sequence as shown in Figure 2.

15. Use in vitro of a nucleic acid according to claim 8, a vector according to claim 9 or 10, a host cell according to claim 11 or 12, a protein according to anyone of claims 1 to 7 or 14 for the induction of apoptosis.

16. Use according to claim 15 wherein said apoptosis is p53-independent.

17. Use according to claim 15 or 16 wherein said apoptosis is tumor-specific.

18. Use according to anyone of claims 15 to 17 wherein said apoptosis is caspase-3 independent.

19. Use according to anyone of claims 15 to 18 further comprising use of a nucleic acid encoding Apoptin or a functional fragment thereof.

20. A pharmaceutical composition comprising a nucleic acid according to claim 8, a vector according to claim 9 or 10, a host cell according to claim 11 or 12, a protein according to anyone of claims 1 to 7 or 14

21. A pharmaceutical composition according to claim 20 further comprising a nucleic acid encoding Apoptin or a functional fragment thereof.

22. A pharmaceutical composition according to claim 20 or 21 for the induction of apoptosis.

23. A pharmaceutical composition according to claim 22 wherein said apoptosis is p53-independent.

24. A pharmaceutical composition according to claim 22 or 23 wherein said apoptosis is tumor-specific.

25. A pharmaceutical composition according to anyone of claims 22 to 24 wherein said apoptosis is caspase-3 independent.

26. A pharmaceutical composition according to anyone of claims 20 to 25 for the treatment of a disease where enhanced cell proliferation or decreased cell death is observed.

27. A pharmaceutical composition according to claim 26 wherein said disease comprises cancer or auto-immune disease.

28. Use of a nucleic acid according to claim 8, a vector according to claim 9 or 10, a host cell according to claim 11 or 12, a protein according to anyone of claims 1 to 7 or 14 for the preparation of a pharmaceutical composition for the treatment of a disease where enhanced cell proliferation or decreased cell death is observed.

29. A vehicle for delivering a nucleic acid of interest to a target cell, said vehicle further comprising a protein according to anyone of claims 1 to 7 or 14.

30. A vehicle according to claim 29 further comprising Apoptin or functional fragment thereof.

31. A conjugate for targeted tumor therapy comprising a targeting moiety having binding affinity for a molecule associated mainly, but not exclusively with the surface of a tumor cell and a protein according to anyone of claims 1 to 7 or 14.

32. A conjugate according to claim 31 further comprising Apoptin or functional fragment thereof.

33. A vehicle delivering a tumoricidal substance or a gene encoding a tumoricidal substance to a molecule associated mainly but not exclusively with a tumor cell, **characterised in that** the tumoricidal substance is a protein according to anyone of claims 1 to 7 or 14.

34. A vehicle according to claim 33 further comprising Apoptin or a gene encoding Apoptin or a functional fragment thereof.

35. A protein according to anyone of claims 1 to 7 or 14 or a functional fragment thereof wherein the fragment has the activing of the amino acid sequence shown in Figure 2 for use in a method of eliminating cells of a target cell population whereby the method is mainly, but not entirely specific for the cells of the target population, whereby a protein according to anyone of claims 1 to 7 or 14 is the cytotoxic agent.

36. A protein according to anyone of claims 1 to 7 or 14 or a functional fragment thereof wherein the fragment has the activing of the amino acid sequenve shown in Figure 2 for use in a method of eliminating cells of a target cell population whereby the cells of said population are not sensitive to other apoptosis inducing agents

37. A protein according to claim 35 or 36 further comprising Apoptin or a functional fragment thereof.

## Patentansprüche

1. Apoptose induzierendes Protein, wobei das Protein die Aminosäuresequenz, wie in der Figur 2 gezeigt, umfasst, oder ein funktionelles Fragment davon, wobei das Fragment die Funktion der Aminosäuresequenz, wie gezeigt in Figur 2, besitzt.

2. Protein gemäß Anspruch 1, fähig zur Vorsehung von Apoptose, ferner in Kombination mit einer anderen Apoptose induzierenden Substanz.

3. Protein gemäß Anspruch 2, wobei die Apoptose induzierende Substanz Apoptin oder ein funktionelles Fragment davon umfasst.

4. Protein gemäß mindestens einem der Ansprüche 1 bis 3, das zur Vorsehung von Apoptose unabhängig von p53 in der Lage ist.

5. Protein gemäß mindestens einem der Ansprüche 1 bis 4, das zur Vorsehung von tumor-spezifischer Apoptose in der Lage ist.

6. Protein gemäß mindestens einem der Ansprüche 1 bis 5, das zur Vorsehung von Apoptose unabhängig von Caspase-3 in der Lage ist.

7. Protein gemäß mindestens einem der Anspruche 1 bis 6, umfassend ein Zellkern-Lokalisierungs-Signal und/oder ein Zellkern-Export-Signal.

8. Isolierte oder rekombinante Nukleinsäure, oder funktionelles Fragment davon, von welcher ein Protein gemäß mindestens einem der Ansprüche 1 bis 7 transkribiert und translatiert werden kann

9. Vektor, umfassend eine Nukleinsäure gemäß Anspruch 8.

10. Vektor gemäß Anspruch 9, umfassend ein Gen-Zuführungs-Vehikel.

11. Wirtszelle, umfassend eine Nukleinsäure gemäß Anspruch 8, welche einen Vektor gemäß Anspruch 9 oder 10 trägt.

12. Wirtszelle gemäß Anspruch 11, welche eine eukaryotische Zelle ist, wie eine Hefezelle oder eine Wirbeltierzelle.

13. Isolierter oder synthetischer Antikörper, der spezifisch ein Protein oder ein funktionelles Fragment davon gemäß mindestens einem der Ansprüche 1 bis 7 erkennt.

14. Protein, spezifisch erkennbar durch einen Antikörper gemäß Anspruch 13, wobei das Protein die Funktion der Aminosäuresequenz, wie gezeigt in Figur 2, besitzt.

15. In-vitro-Verwendung einer Nukleinsäure gemäß Anspruch 8, eines Vektors gemäß Anspruch 9 oder 10, einer Wirtszelle gemäß Anspruch 11 oder 12, eines Proteins gemäß mindestens einem der Ansprüche 1 bis 7 oder 14 für die Induktion von Apoptose.

16. Verwendung gemäß Anspruch 15, wobei die Apoptose unabhängig von p53 ist.

17. Verwendung gemäß Anspruch 15 oder 16, wobei die Apoptose tumorspezifisch ist.

18. Verwendung gemäß mindestens einem der Ansprüche 15 bis 17, wobei die Apoptose unabhängig von Caspase-3 ist.

19. Verwendung gemäß mindestens einem der Ansprüche 15 bis 18, ferner umfassend die Verwendung einer Nukleinsäure, welche Apoptin oder ein funktionelles Fragment davon codiert.

20. Pharmazeutische Zusammensetzung, umfassend eine Nukleinsäure gemäß Anspruch 8, einen Vektor gemäß Anspruch 9 oder 10, eine Wirtszelle gemäß Anspruch 11 oder 12, ein Protein gemäß mindestens einem der Anspruche 1 bis 7 oder 14.

21. Pharmazeutische Zusammensetzung gemäß Anspruch 20, ferner umfassend eine Nukleinsäure, welche Apoptin oder ein funktionelles Fragment davon codiert.

22. Pharmazeutische Zusammensetzung gemäß Anspruch 20 oder 21 für die Induktion von Apoptose.

23. Pharmazeutische Zusammensetzung gemäß Anspruch 22, wobei die Apoptose unabhängig von p53 ist.

24. Pharmazeutische Zusammensetzung gemäß Anspruch 22 oder 23, wobei die Apoptose tumorspezifisch ist.

25. Pharmazeutische Zusammensetzung gemäß mindestens einem der Ansprüche 22 bis 24, wobei die Apoptose unabhängig von Caspase-3 ist.

26. Pharmazeutische Zusammensetzung gemäß mindestens einem der Ansprüche 20 bis 25 für die Behandlung einer Krankheit, bei der erhöhte Zellproliferation oder verminderter Zelltod beobachtet werden.

27. Pharmazeutische Zusammensetzung gemäß Anspruch 26, wobei die Krankheit Krebs oder Autoimmun-Krankheit umfasst.

28. Verwendung einer Nukleinsäure gemäß Anspruch 8, eines Vektors gemäß Anspruch 9 oder 10, einer Wirtszelle gemäß Anspruch 11 oder 12, eines Proteins gemäß mindestens einem der Ansprüche 1 bis 7 oder 14, für die Herstellung einer pharmazeutischen Zusammensetzung für die Behandlung einer Krankheit, bei der erhöhte Zellproliferation oder verminderter Zelltod beobachtet werden.

29. Vehikel zur Zuführung einer Nukleinsäure von Interesse an eine Zielzelle, wobei das Vehikel ferner ein Protein gemäß mindestens einem der Ansprüche 1 bis 7 oder 14 umfasst.

30. Vehikel gemäß Anspruch 29, ferner umfassend Apoptin oder ein funktionelles Fragment davon.

31. Konjugat für die zielgerichtete Tumortherapie, umfassend eine Targeting-Einheit mit Bindungsaffinität für ein Molekül, das hauptsächlich, aber nicht ausschließlich, mit der Oberfläche einer Tumorzelle assoziiert ist, und ein Protein gemäß mindestens einem der Ansprüche 1 bis 7 oder 14.

32. Konjugat gemäß Anspruch 31, ferner umfassend Apoptin oder ein funktionelles Fragment davon.

33. Vehikel, welches eine tumorabtötende Substanz oder ein Gen, codierend eine tumorabtötende Substanz, an ein Molekül, das hauptsächlich, aber nicht ausschließlich, mit einer Tumorzelle assoziiert ist, zuführt, **dadurch gekennzeichnet dass** die tumorabtötende Substanz ein Protein gemäß mindestens einem der Ansprüche 1 bis 7 oder 14 ist.

34. Vehikel gemäß Anspruch 33, ferner umfassend Apoptin oder ein Apoptin codierendes Gen oder ein funktionelles Fragment davon.

35. Protein gemäß mindestens einem der Ansprüche 1 bis 7 oder 14 oder ein funktionelles Fragment davon, wobei das Fragment die Aktivität der in Figur 2 gezeigten Aminosäuresequenz besitzt, zur Verwendung in einem Verfahren zum Eliminieren von Zellen einer Zielzellpopulation, wobei das Verfahren hauptsächlich, aber nicht vollkommen, spezifisch für die Zellen der Zielpopulation ist, wobei ein Protein gemäß mindestens einem der Ansprüche 1 bis 7 oder 14 das cytotoxische Mittel ist.

36. Protein gemäß mindestens einem der Ansprüche 1 bis 7 oder 14 oder ein funktionelles Fragment davon, wobei das Fragment die Aktivität der in Figur 2 gezeigten Aminosäuresequenz besitzt, zur Verwendung in einem Verfahren zum Eliminieren von Zellen einer Zielzellpopulation, wobei die Zellen der Population nicht empfindlich gegenüber anderen Apoptose induzierenden Mitteln sind.

37. Protein gemäß Anspruch 35 oder 36, ferner umfassend Apoptin oder ein funktionelles Fragment davon

## Revendications

1. Protéine induisant l'apoptose, dans laquelle ladite protéine comprend la séquence d'acide aminé montrée à la figure 2 ou un fragment fonctionnel de celle-ci, dans laquelle ledit fragment a la fonction de la séquence d'acide aminé telle que montrée à la figure 2.

2. Protéine selon la revendication 1, capable de fournir une apoptose en combinaison avec une autre substance induisant l'apoptose.

3. Protéine selon la revendication 2, dans laquelle ladite substance induisant l'apoptose comprend l'apoptine ou un fragment fonctionnel de celle-ci.

4. Protéine selon l'une quelconque des revendications 1 à 3, capable de fournir l'apoptose indépendante de p53.

5. Protéine selon l'une quelconque des revendications 1 à 4, capable de fournir une apoptose spécifique aux tumeurs.

6. Protéine selon l'une quelconque des revendications 1 à 5, capable de fournir une apoptose indépendante de la caspase-3.

7. Protéine selon l'une quelconque des revendications 1 à 6, comprenant un signal de location nucléaire et/ou un signal de transport nucléaire.

8. Un acide nucléique isolé ou recombinant ou un fragment fonctionnel de celui-ci à partir duquel une protéine selon l'une quelconque des revendications 1 à 7 peut être transcrite et traduite.

9. Un vecteur comprenant un acide nucléique selon la revendication 8.

10. Un vecteur selon la revendication 9 comprenant un véhicule de transport de gènes.

11. Une cellule hôte comprenant un acide nucléique selon la revendication 8 contenant un vecteur selon les revendications 9 ou 10.

12. Une cellule hôte selon la revendication 11, qui est une cellule eucaryote telle qu'une cellule de levure ou une cellule de vertébré.

13. Un anticorps isolé ou synthétique reconnaissant spécifiquement une protéine ou un fragment fonctionnel de celle-ci, selon l'une quelconque des revendications 1 à 7.

14. Une protéine spécifiquement reconnaissable par un anticorps selon la revendication 13, dans laquelle ladite protéine a la fonction de la séquence d'acide aminé montrée à la figure 2.

15. Utilisation in vitro d'un acide nucléique selon la revendication 8, d'un vecteur selon la revendication 9 ou 10, d'une cellule hôte selon la revendication 11 ou 12, d'une protéine selon l'une quelconque des revendications 1 à 7 ou 14 pour induire l'apoptose.

16. Utilisation selon la revendication 15, dans laquelle ladite apoptose est indépendante-p53.

17. Utilisation selon l'une quelconque des revendications 15 ou 16, dans laquelle ladite apoptose est spécifique aux tumeurs.

18. Utilisation selon l'une quelconque des revendications 15 à 17, dans laquelle ladite apoptose est indépendante de la caspase-3.

19. Utilisation selon l'une quelconque des revendications 15 à 18, comprenant en outre l'utilisation d'un acide nucléique codant pour la protéine ou un fragment fonctionnel de celui-ci.

20. Une composition pharmaceutique comprenant un acide nucléique selon la revendication 8, un vecteur selon la revendication 9 ou 10, une cellule hôte selon la revendication 11 ou 12, une protéine selon l'une quelconque des revendications 1 à 7 ou 14.

21. Une composition pharmaceutique selon la revendication 20, comprenant en outre un acide nucléique codant pour l'apoptine ou un fragment fonctionnel de celui-ci.

22. Une composition pharmaceutique selon la revendication 20 ou 21 pour l'induction de l'apoptose.

23. Une composition pharmaceutique selon la revendication 22, dans laquelle ladite apoptose est indépendante-p53.

24. Une composition pharmaceutique selon la revendication 22 ou 23, dans laquelle ladite apoptose est spécifique aux tumeurs.

25. Une composition pharmaceutique selon l'une quelconque des revendications 22 à 24, dans laquelle ladite apoptose est indépendante de la caspase-3.

26. Une composition pharmaceutique selon l'une quelconque des revendications 20 à 25 pour le traitement d'une maladie dans laquelle la prolifération cellulaire accrue ou la mort cellulaire décrue est observée.

27. Une composition pharmaceutique selon la revendication 26, dans laquelle ladite maladie comprend le cancer ou une maladie auto-immune.

28. Utilisation d'un acide nucléique selon la revendication 8, d'un vecteur selon la revendication 9 ou 10, d'une cellule hôte selon la revendication 11 ou 12, d'une protéine selon l'une quelconque des revendications 1 à 7 ou 14 pour la préparation d'une composition pharmaceutique pour le traitement d'une maladie dans laquelle la prolifération cellulaire accrue ou la mort cellulaire décrue est observée.

29. Un véhicule pour transporter un acide nucléique d'intérêt à une cellule cible, ledit véhicule comprenant en outre une protéine selon l'une quelconque des revendications 1 à 7 ou 14.

30. Un véhicule selon la revendication 29 comprenant en outre l'apoptine ou un fragment fonctionnel de celle-ci.

31. Un conjugué pour la thérapie ciblée de tumeur comprenant un fragment de ciblage ayant une affinité de liaison pour une molécule associée essentiellement mais pas exclusivement avec la surface d'une cellule tumorale et une protéine selon l'une quelconque des revendications 1 à 7 ou 14.

32. Un conjugué selon la revendication 31 comprenant en outre l'apoptine ou un fragment fonctionnel de celle-ci.

33. Un véhicule transportant une substance destructrice de tumeur ou un gène codant pour une substance destructrice de tumeur à une molécule associée essentiellement mais pas exclusivement avec une cellule tumorale, **caractérisé en ce que** la substance destructrice de tumeur est une protéine selon l'une quelconque des revendications 1 à 7 ou 14.

34. Un véhicule selon la revendication 33 comprenant en outre l'apoptine ou un gène encodant l'apoptine ou un fragment fonctionnel de celle-ci.

35. Une protéine selon l'une quelconque des revendications 1 à 7 ou 14 ou un fragment fonctionnel de celle-ci dans laquelle le fragment a l'activité de la séquence d'acide aminé montrée à la figure 2 pour une utilisation dans une méthode pour éliminer les cellules d'une population de cellules cibles dans laquelle la méthode est essentiellement mais pas exclusivement spécifique pour les cellules de la population cible, dans laquelle une protéine selon l'une quelconque des revendications 1 à 7 ou 14 est l'agent cytotoxique.

36. Une protéine selon l'une quelconque des revendications 1 à 7 ou 14 ou un fragment fonctionnel de celle-ci dans laquelle le fragment a l'activité de la séquence d'acide aminé montrée à la figure 2 pour une utilisation dans une méthode pour l'élimination de cellules d'une population de cellules cibles dans laquelle les cellules de ladite population ne sont pas sensibles à d'antres agents induisant l'apoptose.

37. Une protéine selon la revendication 35 ou 36, comprenant en outre l'apoptine ou un fragment fonctionnel de celle-ci.
